# EUROPEAN PATENT SPECIFICATION

(11) **EP 3 197 702 B1**
(45) Date of publication and mention of the grant of the patent: **18.12.2019**
(21) Application number: 15732337.9
(22) Date of filing: 24.04.2015
(51) Int. Cl.: B60K 28/06, B60K 28/04, B60K 28/02, B60W 40/08

(54) **A SYSTEM FOR DETECTING THE EXISTENCE OF ILLEGAL ACTIVE SUBSTANCES IN A DRIVER OF A VEHICLE**
EIN SYSTEM ZUR ERFASSUNG DES VORLIEGENS VON ILLEGALEN WIRKSTOFFEN IN EINEM FAHRER EINES FAHRZEUGS
SYSTÈME DE DÉTECTION DE L'EXISTENCE DE SUBSTANCES ACTIVES ILLÉGALES CHEZ UN CONDUCTEUR DE VÉHICULE

(43) Date of publication of application: 02.08.2017
(73) Proprietor: Cristofaro, Sergio, 80078 Pozzuoli Naples (IT); Netcom Industry S.r.l., 80122 Napoli (IT)
(72) Inventor: Cristofaro, Sergio, 80078 Pozzuoli (NA) (IT)
(74) Representative: Cardelli, Guido
(86) International application number: PCT/IB2015/053011
(87) International publication number: WO 2015/162598

(56) References cited:
- EP-A1- 1 703 045
- WO-A1-2013/132521
- CN-A- 102 303 529
- CN-A- 103 129 389
- US-A1- 2008 316 037
- US-A1- 2010 010 689

## Description

The present invention relates to a system to increase safety for drivers and, more specifically, to a system to detect and measure the possible consumption of illegal active substances in a driver of a vehicle.

### State of the art

As it is known, in recent years, the regulations have modified the maximum limit of alcohol content in a body of a driver (a.k.a. BAC), leading to values every time lower and with the aim to safeguard both the safety on roads and for the driver and other people.

In this sense, there have been proposed a number of systems and/or devices to increase the driver's safety in case of the driver be in a physical and/or psychological condition that it is not suitable such as, for example, a state of intoxication or during use of drugs.

For example, from WO2013132521A1 it is known a first type of device to be mounted on the vehicle and which detects the blood alcohol level (hereinafter referred to as BAC) of an individual via exhalation of the driver inside a analysis port of the device (i.e., detection of the alcohol content in the breath). According to this type of device when it detects a BAC value higher than the legal limit, the device shuts down the vehicle until a new measurement/value falls within the established legal limits.

This solution, even if effective from the practical point of view because it is extremely simple, has however the drawback of not constraining the analysis of the breath for the effective/actual driver, as it is possible to exhale into the device from a different person other than the driver. Hence, the result is a lack of efficacy for the actual safety, given the opportunity to "cheat" the device.

Furthermore, it is known from US2004083031 and US2008316037A1 a system for detecting and measuring the BAC of a driver, which system comprises a series of sensing devices inserted inside the vehicle and which include one or more sensing devices of the BAC of a driver by means of sensors which sense via contact of the fingers or the palm of the hands of the driver, and which sensors are arranged inside in a coating on the steering wheel of the vehicle. The sensors are connected to a control unit, which depending on the signal from the sensors causes the disabling of the operation of the vehicle. The system can also include one or more information displays and / or lights to inform the driver of the present condition.

This system albeit precise has a first drawback due to the fact that it appears very complex in its interaction / management, and it is subject to possible malfunctions due to the arrangement of the BAC sensors onto the steering wheel, which are extremely exposed to both environmental and mechanical stresses during the use of the steering wheel itself, said stresses inducing calibration failures, malfunction, and / or breakage of the BAC sensors. Moreover, there is another drawback which is given by the fact that in case of malfunction is not provided an "override" of the system by the user, with consequent impossibility to use the vehicle. Additionally, since all the sensors of the system are fixedly mounted inside the vehicle, the replacing of each of them entails a high cost of intervention and stop of the vehicle.

In addition, in the Japanese document Patent Application JP2008132961 it is known a system mounted in a vehicle and which it is capable of inhibiting the driving of the vehicle when the driver is under the alcohol influence. According to this document, the system provides the control of the vehicle as a result of the sensing and the measurement of the BAC of a driver through analysis of his breath and / or BAC sensors arranged on the steering wheel. Consequently to the measurement of the BAC of the driver, it is checked the light transmission factor of the crystals of the vehicle (making them more or less opaque to light), both the position of the vehicle, both its speed. In this condition, the vehicle may ignite but cannot move. Furthermore, there are provided a series of light signals and radio frequency information both in the vehicle and outside of the same, with the purpose to inform both the police and other drivers of the condition of the driver of the vehicle.

Nevertheless, this system has a first drawback due to the fact that it appears very complex in its interaction / management, as well as in the case of malfunction it is not provided an "override" of the system by the user, with consequent impossibility to use the vehicle. Another drawback is the fact that all the sensors of the system are mounted in the vehicle, and thus their replacement due to malfunction involves a high cost of intervention and stop of the vehicle.

On the other hand, from EP1703045A1 it is known a vehicle key arrangement capable of preventing the drunken driving. The vehicle key arrangement comprises: a key portion with the forward end portion thereof insertable into the key hole of the vehicle such as a train, a motorbike and an automobile, a holder portion arranged at the base end portion of the key portion, a projection member projected from the holder portion along the key portion, a spring for urging the projection member in the direction of projection, a breath component detection means for detecting the alcohol contained in the breath of the user, and a lock mechanism for locking the projection member in a projected state in accordance with the detection result of the breath component detection means.

According to this document, the user has to breath on or touch said breath component detection means, and only upon consent of said breath component detection means the functioning of the key portion and vehicle it is enabled.

Although functional said key arrangement has the drawback of being extremely complex inside since housing a number of electronic and mechanical devices, and therefore also being extremely subject to mechanical/electronic fail.

Therefore, the aim of the present invention it is to solve the above drawbacks by providing a more reliable system for the measuring of the BAC and / or eventual presence of illegal active substances in a driver's blood inside a vehicle, according to claim 1.

According to the system of the present invention, it is provided the synergy of the measuring of the BAC values of the driver, the assessment of the condition of the latter according to the set limits to be not exceeded, the control that be actually the driver to be analysed, and in the case where there are discrepancies or at least one of the detected/sensed values exceed predetermined limits, the stop of the vehicle it is realized until the sensed values from each of the sensors do not fall within predetermined values.

### Brief description of the invention

The object of the present invention it is a system for the measuring/sensing of the BAC and / or possible presence of illegal active substances in a driver of a vehicle, the system comprising:
- A first portable device equipped with at least one sensor of the BAC and / or other illegal active substances of a person under examination; and
- A second device fixedly mounted on the vehicle which is interfaced with said first portable device to receive / send one or more electrical signals to a control unit mounted on said vehicle;
the arrangement being such that said electrical signals from said second device are correlated to the detected/read values of BAC and / or other illegal active substances from said first device, and said control unit being adapted to control the management of the vehicle according to said values of said signals.

According to a first aspect of the system of the present invention, said first portable device comprises at least one sensor for detecting the presence of ethanol from the sweat of the driver's hands and / or a device for detecting the eventual presence of drugs (such as, for example, cannabis, amphetamines, morphine, cocaine) through sweat analysis of the driver's hands.

According to a second aspect of the system of the present invention, it is provided that said first portable device enable access to the vehicle driver only after passing the test for analysis of the presence of illegal substances in the sweat of the hands.

According to a third aspect of the system of the present invention, it is provided that said first device interfaces with said second device inside the vehicle, and said second interface device it is mounted in the vehicle at the dashboard region which is accessible to only the driver of the vehicle when he is sitting in the driving position into the vehicle.

According to a fourth aspect of the system of the present invention, there is also provided a device for detecting the alcohol vapours within the vehicle.

According to a fifth aspect of the system of the present invention, there is also provided a device for detecting and interpreting voice testing on the driver.

According to a sixth aspect of the system of the present invention, it is further provided a device for detecting the presence of the driver's hands on the steering wheel.

Therefore, the present invention provides a system for detecting the existence of illegal active substances in a driver of a vehicle according to the appended claims.

### Detailed description of the invention

A detailed description of a preferred embodiment of the system for detecting the existence of illegal active substances in a driver of a vehicle of the present invention will now be given, by way of a non limiting example, and making reference to the appended drawings, wherein:
Figure 1 is a perspective view that illustrates a first portable device according to the system of the present invention during its use;
Figure 2 is a schematic view illustrating a passenger compartment and a dashboard of a vehicle which incorporates the system of the present invention;
Figure 3 is a schematic view illustrating the position of the driver during operation of the system and the detection of the BAC according to the present invention;
Figure 4 is a schematic view illustrating the steering wheel which incorporates a device for detection of presence according to the system of the present invention;
Figure 5 is a schematic view that partially illustrates the position of the hands of a driver on a steering wheel while driving; and
The 6 is a schematic view illustrating the position of a driver's hands during detection of the device according to the present invention.

With reference now to the figures it will be described the operation of each device of the system of the present invention and their mutual interfacing.

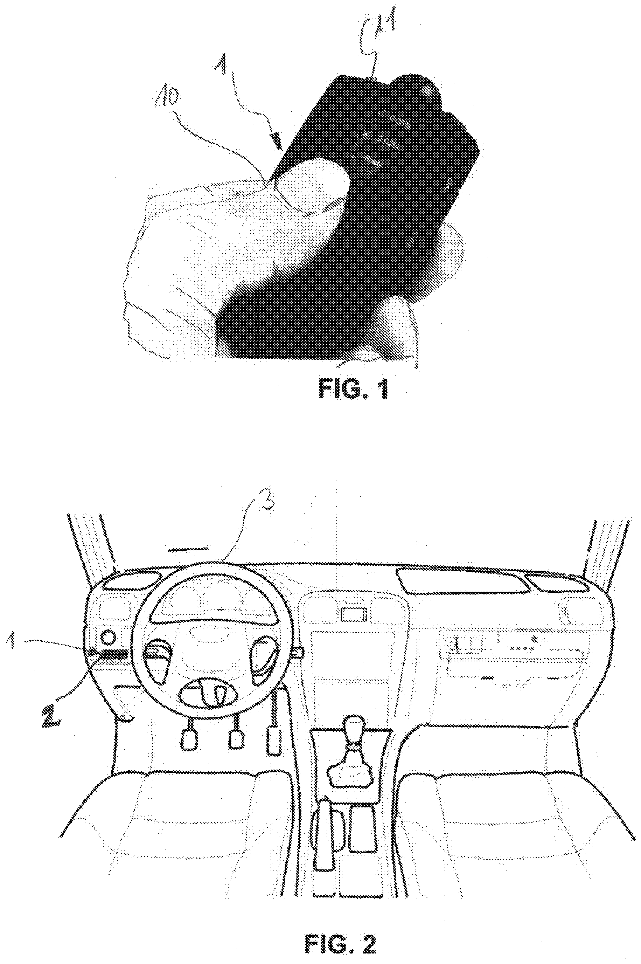

Figure 1 illustrates a first portable device which comprises a body 1 which encloses at least one sensor for detecting the BAC and / or other illegal active substances existing in the sweat of the hands of a person concerned. With this aim, on the body 1 it is formed a detection surface 10 adapted to provide the sweat analysis surface for the driver when he holds with his fingers said surface.

According to the system, it is sufficient that the user grasps the device for a period of time established (and necessary to perform the analysis by the sensor). Following the analysis, the result (positive or negative) it will be indicated by light devices 11 on the main body 1.

Only in case of positive outcome of the analysis, the portable device 1 enables the opening of the doors or the ignition of the vehicle (the latter not shown).

With reference now to Figure 2 there is illustrated a dashboard of a vehicle which incorporates the system of the present invention. According to the system, there is provided a second device 2 for interfacing with the first portable device 1, the second interfacing device 2 it is mounted at a dashboard region which is accessible only to the driver of the vehicle while he is in the driving position, i.e. at the part of the dashboard which is provided between the steering wheel 3 and the vehicle's door (the latter not shown in the figure).

The second interface device 2 provides a region for housing the first portable device 1 according to a "docking" type connection. More precisely, the system of the present invention provides that the portable device 1 interfaces with the device 2 via a "contactless" technology such as, for example, transponder, or Bluetooth, or the like.

In this way it is possible to transmit data to / from the first device 1 and the second device 2.

The system of the present invention also provides a control unit downstream of the second device and connected with the latter which control and manages all the information from all the devices of the system of the present invention, and at the same time it controls the management of the vehicle (better illustrated below).

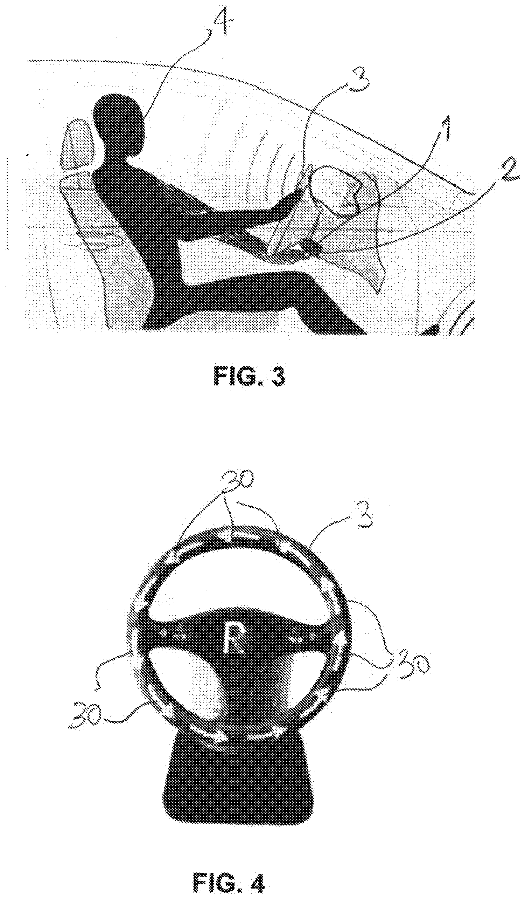

With reference now to Figure 3, there is illustrated the operation between the first portable device 1, the second interface device 2 and a vehicle's driver 4.

More precisely, it is expected that even while driving the driver 4 can access the first portable device 1 in its housing interfacing 2. In fact, the arrangement is such that the driver 4 while driving can easily reach with the fingers of the hand the portable device 1 even if the other hand is engaged on the steering wheel 3.

It will be immediately obvious to those skilled in the relevant technical field that under this provision, it is possible to make an analysis on the driver while driving. In fact, in this way it can be performed an analysis on the driver of the values of the BAC and / or other substances even while driving, and transmitting the relevant data to the control unit of the vehicle directly from the portable device 1 via the second interfacing device 2.

Furthermore, according to the present invention it is provided that the system will conduct a repeated analysis on the driver during the driving and after passing the first check before boarding onto the vehicle.

With the above aim and with reference now to Figure 4, according to the system of the present invention it is provided that on the steering wheel 3 there are provided a number of sensing regions 30 (via an impedance meter) each adapted to detect the physical presence of the hand of the driver onto the steering wheel 3 in the manner already known in the art.

It should here be specified that the detection of the presence of a driver's hand onto the steering wheel 3 while driving it is crucial for providing the enabling signal to the control unit of the actual presence of the driver during the analysis in motion on the first portable device 1. More precisely, according to the system of the present invention it is provided that the control unit has to check the effective presence of the driver during the analysis by executing the control of the presence of the hand on the steering wheel 3 and the other hand on the portable device 1.

In this manner it is practically impossible to "trick" the control unit, i.e. in the case in which the driver's hand under analysis be replaced by another person (not intoxicated) and during the analysis and the driving simultaneously.

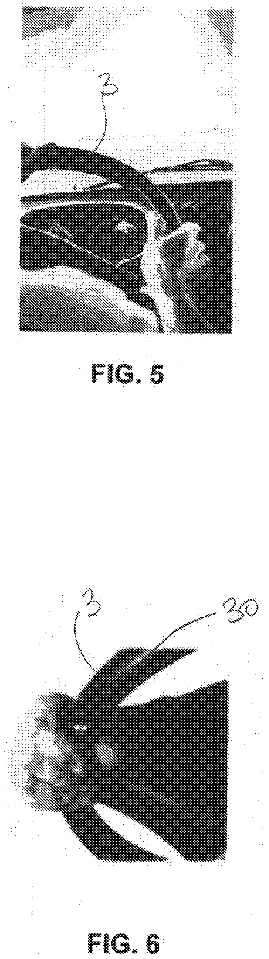

With reference now to Figures 5 and 6 there is illustrated the position of the driver's hands while driving and during analysis, respectively.

The sensing/metering regions 30 are arranged circumferentially on the entire surface of the steering wheel 3 (front and rear) and in such a manner as to ensure a reliable reading even with a single hand, when the latter grasps the steering wheel 3.

Further to the above illustrated sensing/analysing devices, the system off the present invention can further include the following devices or sensors arranged in the vehicle:
- Driver detecting sensor onto the driver's seat;
- Sensor for detecting the position of the hands on the steering wheel 3;
- Detection sensor of the BAC inside the cabin and at the driving position (i.e., sampling of the air inside the cabin from the front to the driving position);
- Voice sensor for detecting the driver's voice; and
- Sensor for detecting of muscle strength of the hands on the steering wheel (hand grip test).

### Operation

### Detecting the presence of the actual driver

According to the invention, it is provided that the system first verifies the presence of the actual driver in the driving seat by detecting the presence on the seat by means of position or presence sensor on the seat (for example, the sensors already used for the alarm safety belts not connected). In this case, he should not raise from the seat during the entire analysis cycle.

Once verified the presence of the driver 4 at the driving position, the system initiates an audible message indicating the start of the sequenced analysis for the detection of BAC and / or other toxic substances via the first portable device 1 inserted in the second interfacing device 2 (Figure 3).

During this analysis, the driver 4 must sit and with the other hand (i.e., the hand not been under analysis) must hold the steering wheel 3 and never detach from the latter.

Furthermore, it must be highlighted here that for this type of analysis the driver cannot wear any kind of coverage of the hands such as gloves, since this would lead to an error signal to the sensors and by stopping the analysis procedure, and reiterating the starting of the same procedure until it is detected the correct value of the signal coming from the sensing regions 30 onto the steering wheel 3.

Similarly, also for the proper operation of the first portable device 1 it is not possible to wear gloves, since it would result in an error signal which would invalidate the operation of the whole system, reiterating the starting procedure of analysis until a correct value to be analysed is read by the sensor.

Furthermore, in the case where it is provided the system can include a fingerprint recognition of the driver in such a way as to oblige the latter to do not wear gloves or similar, in order to not invalidating the procedure.

### Detection of alcohol vapours in the passenger

The driver is located frontally to the steering wheel (figure 3). In the steering wheel 3 or at the front location of the dashboard it is arranged a sensor for detecting the concentration of alcohol vapours coming from the driver's position. Upon detecting a value which passes established limits there is provided an acoustic and / or light alarm to the driver.

### Detection and interpretation of the test voice driver

The system may include in the management of the control unit also a further procedure where to the driver are imposed with periodic time lapse voice tests where are supplied simple questions to which he must answer.

The system will be able to also detect alterations of the voice or erroneous answers. In this condition, the system sends an alarm to the driver asking it to stop the vehicle for not having passed the test voice.

### Detection of the strength and hands coordination onto the steering wheel (hand grip test)

It can be provided onto the whole circumference of the steering wheel 3 further sensors to detect the pressure exerted by the fingers and hands (i.e., muscle strength and coordination of the hands) on the steering wheel 3 (hand grip test) in conjunction with specific voice instructions (voice test).

So, while driving and only after having passed all the analysis / control procedures the driver can have a confirmation from the control unit to continue driving the vehicle. That is to say that even in the case of only one of the devices gives a negative result, it will be immediately commenced by the control unit a reiteration procedure of analysis and control to the driver, and as a consequence of a further fail of the same procedure, the vehicle will be gradually stopped.

According to the system of the present invention, it is provided that during the driving it is performed a cycle of analysis of all the different sensors established time periods (for example, every 5 or 10 minutes). Accordingly, it can be ensured that the driver be always under control even if he has passed the last test.

While driving, if a change in these parameters has been detected and compared with the previous analysed values and resulting outside the established limits according to the law, the system will let the vehicle slow down until it stops gradually in order to do not put in a danger condition the driver and the passengers.

Finally, the system provides an "emergency" deactivation device for deactivating the control system and in order to allow the starting of the vehicle even without having to undergo the aforementioned tests routine to the driver, for example, in the case of serious emergencies that may occur, such as the need to use the vehicle in the event of fire, of aggression by others, or illnesses to some other person to be rescued. Said emergency device will be lifesaving but directly connected to an "operations center" such as police or the like, that will identify what happened and allow the use of the vehicle for a short period of time (few minutes) and only on verbal directions of operators trained to provide information about.

This automatic connection to a "control operational centre" will guarantee the use of the system even when disabled but under penalty fines in the case of misuse.

## Claims

1. A system for the measuring of the blood alcohol content in a body of a driver (i.e., BAC) and/or possible existence of illegal active substances in a body of a driver (4) of a vehicle comprising a steering wheel (3), a dashboard, and a control unit, comprising:
- a first portable device (1) which comprises at least one sensor for detecting the BAC and / or other illegal active substances of said driver (4) ;
- a second interfacing device (2) fixedly mounted on the vehicle, and which it is interfaced with said first portable device (1) to receive / send one or more electrical signals to a control unit, the latter being mounted on said vehicle; the arrangement being such that said electrical signals from said second device (2) are related to the detected / sensed values of BAC and / or other illegal active substances and coming from said first device (1), and said control unit being adapted to control the management of the whole vehicle consequently and according to the values of said electrical signals;
**characterized in that**
- said first portable device (1) enables access of the driver (4) to the vehicle only on passing the analysis test of BAC and / or the presence of illegal substances in his sweat of the hands;
- wherein said second interface device (2) comprises a region for housing or docking said first portable device (1); and
- wherein the system is configured to run a reiterated analysis on the driver even during the driving and subsequently to the first analysis before boarding the vehicle.

2. System for the measuring of the BAC and / or possible presence of illegal active substances in a driver (4) of a vehicle according to claim 1, wherein said first device (1) interfaces with said second device (2) inside the vehicle, and said second interface device (2) it is mounted in the vehicle at a region of the vehicle dashboard which is accessible to only the driver (4) of the vehicle while the latter is in the driving sitting condition.

3. System for the measuring of the BAC and / or possible presence of illegal active substances in a driver (4) of a vehicle according to any of the preceding claims, further comprising a voice and interpretation sensing device for the voice testing on the driver (4).

4. System for the measuring of the BAC and / or possible presence of illegal active substances in a driver (4) of a vehicle according to any of the preceding claims, further comprising a plurality of detecting device (30) to detect the presence of the driver's hands onto the steering wheel (3).

5. System for the measuring of the BAC and / or possible presence of illegal active substances in a driver (4) of a vehicle according to any of the preceding claims, wherein said second interface device (2) it is arranged at a region accessible only to the driver (4) while driving, said region being at the part of the dashboard comprised between the steering wheel (3) and the vehicle's door.

6. System for the measuring of the BAC and / or possible presence of illegal active substances in a driver (4) of a vehicle according to any of the preceding claims, wherein the portable device (1) interfaces with the interface device (2) via a contactless technology by a Transponder.

7. System for the measuring of the BAC and / or possible presence of illegal active substances in a driver (4) of a vehicle according to any of claims 1-5, wherein the portable device (1) interfaces with the interface device (2) via a contactless technology.

8. Use of a system for the measuring of the BAC and / or possible presence of illegal active substances in a driver (4) of a vehicle according to any of the preceding claims, wherein during driving the driver (4) interacts with the first portable device (1) through one hand and without removing said portable device (1) from the interface device (2), and at the same time while keeping the other hand engaged on the steering wheel (3).

9. System for the measuring of the BAC and / or possible presence of illegal active substances in a driver (4) of a vehicle according to any of the claims 1-7, wherein said control unit is configured to perform the control of the effective presence of the driver (4) during the analysis by checking the presence of the hand onto the steering wheel (3) and the other hand onto the mobile device (1).

## Patentansprüche

1. System zum Messen des Blutalkoholgehalts im Körper eines Fahrers (d. h. vom BAC) und/oder des möglichen Vorliegens von illegalen Wirkstoffen im Körper eines Fahrers (4) eines Fahrzeugs, umfassend ein Lenkrad (3), ein Armaturenbrett und ein Steuergerät,
umfassend:
- eine erste tragbare Vorrichtung (1), die mindestens einen Sensor zur Erfassung des BAC und/oder sonstiger illegaler Wirkstoffe des Fahrers (4) umfasst;
- eine zweite Schnittstellenvorrichtung (2), die fix am Fahrzeug montiert ist und mit der ersten tragbaren Vorrichtung (1) verbunden ist, um ein oder mehrere elektrische Signale von einem Steuergerät zu empfangen/an dieses zu übermitteln, das am Fahrzeug montiert ist,
wobei die Anordnung so ausgelegt ist, dass die elektrischen Signale von der zweiten Vorrichtung (2) mit den erfassten/gemessenen Werten des BAC und/oder anderer illegaler Wirkstoffe, die von der ersten Vorrichtung (1) eingehen, verknüpft sind, und wobei das Steuergerät ausgelegt ist, um das Management des gesamten Fahrzeugs entsprechend und gemäß den Werten dieser elektrischen Signale zu steuern,
**dadurch gekennzeichnet, dass**
- die erste tragbare Vorrichtung (1) dem Fahrer (4) den Zugang zum Fahrzeug nur dann erlaubt, wenn dieser den Analysetest in Bezug auf den BAC und/oder das Vorliegen von illegalen Stoffen im Schweiß seiner Hände besteht,
- wobei die zweite Schnittstellenvorrichtung (2) eine Region zur Unterbringung oder zum Andocken der ersten tragbaren Vorrichtung (1) umfasst und
- wobei das System ausgelegt ist, um eine wiederholte Analyse des Fahrers auch während der Fahrt und nach der ersten Analyse vor dem Besteigen des Fahrzeugs durchzuführen.

2. System zum Messen des BAC und/oder des möglichen Vorliegens von illegalen Wirkstoffen in einem Fahrer (4) eines Fahrzeugs nach Anspruch 1, wobei die erste Vorrichtung (1) mit der zweiten Vorrichtung (2) im Fahrzeug verbunden ist und die zweite Schnittstellenvorrichtung (2) im Fahrzeug in einer Region des Armaturenbretts des Fahrzeugs montiert ist, die nur für den Fahrer (4) des Fahrzeugs zugänglich ist, während sich dieser in der sitzenden Fahrposition befindet.

3. System zum Messen des BAC und/oder des möglichen Vorliegens von illegalen Wirkstoffen in einem Fahrer (4) eines Fahrzeugs nach einem der vorhergehenden Ansprüche, zudem umfassend eine Sprach- und Interpretationserfassungsvorrichtung für den Sprachtest des Fahrers (4).

4. System zum Messen des BAC und/oder des möglichen Vorliegens von illegalen Wirkstoffen in einem Fahrer (4) eines Fahrzeugs nach einem der vorhergehenden Ansprüche, zudem umfassend eine Vielzahl an Erfassungsvorrichtungen (30), um zu erfassen, ob sich die Hände des Fahrers auf dem Lenkrad (3) befinden.

5. System zum Messen des BAC und/oder des möglichen Vorliegens von illegalen Wirkstoffen in einem Fahrer (4) eines Fahrzeugs nach einem der vorhergehenden Ansprüche, wobei die zweite Schnittstellenvorrichtung (2) in einer Region angeordnet ist, die während der Fahrt nur für den Fahrer (4) zugänglich ist, wobei sich diese Region in einem Teil des Armaturenbretts zwischen dem Lenkrad (3) und der Fahrertür befindet.

6. System zum Messen des BAC und/oder des möglichen Vorliegens von illegalen Wirkstoffen in einem Fahrer (4) eines Fahrzeugs nach einem der vorhergehenden Ansprüche, wobei die tragbare Vorrichtung (1) mit der Schnittstellenvorrichtung (2) mittels berührungsloser Technologie durch einen Transponder verbunden ist.

7. System zum Messen des BAC und/oder des möglichen Vorliegens von illegalen Wirkstoffen in einem Fahrer (4) eines Fahrzeugs nach einem der Ansprüche 1-5, wobei die tragbare Vorrichtung (1) mit der Schnittstellenvorrichtung (2) mittels berührungsloser Technologie verbunden ist.

8. Verwendung eines Systems zum Messen des BAC und/oder des möglichen Vorliegens von illegalen Wirkstoffen in einem Fahrer (4) eines Fahrzeugs nach einem der vorhergehenden Ansprüche, wobei der Fahrer (4) während der Fahrt mit der ersten tragbaren Vorrichtung (1) mittels einer Hand interagiert und ohne die tragbare Vorrichtung (1) von der Schnittstellenvorrichtung (2) zu entfernen, während er gleichzeitig die andere Hand am Lenkrad (3) festhält.

9. System zum Messen des BAC und/oder des möglichen Vorliegens von illegalen Wirkstoffen in einem Fahrer (4) eines Fahrzeugs nach einem der Ansprüche 1-7, wobei das Steuergerät konfiguriert ist, um die Kontrolle der tatsächlichen Anwesenheit des Fahrers (4) während der Analyse durchzuführen, indem die Anwesenheit der Hand am Lenkrad (3) und der anderen Hand an der mobilen Vorrichtung (1) geprüft wird.

## Revendications

1. Système de mesure du taux d'alcool dans le sang dans un corps d'un conducteur (c'est-à-dire le BAC) et/ou de l'existence possible de substances actives illégales dans un corps d'un conducteur (4) de véhicule comprenant un volant (3), un tableau de bord et une unité de commande,
comprenant :
- un premier dispositif portable (1) comprenant au moins un capteur pour détecter le BAC et/ou d'autres substances actives illégales chez ledit conducteur (4) ;
- un second dispositif d'interfaçage (2), monté fixement sur le véhicule, et étant interfacé avec ledit premier dispositif portable (1) pour recevoir / envoyer un ou plusieurs signaux électriques à une unité de commande, cette dernière étant montée sur ledit véhicule ;
la disposition étant telle que lesdits signaux électriques provenant dudit second dispositif (2) concernent les valeurs détectées / captées du BAC et/ou d'autres substances actives illégales et provenant dudit premier dispositif (1), et ladite unité de commande étant adaptée pour commander la gestion de l'ensemble du véhicule en conséquence et selon les valeurs desdits signaux électriques ;
**caractérisé en ce que**
- ledit premier dispositif portable (1) permet l'accès du conducteur (4) au véhicule uniquement s'il a passé le test d'analyse du BAC et/ou de la présence de substances illégales dans la transpiration de ses mains ;
- dans lequel ledit second dispositif d'interface (2) comprend une zone pour loger ou accueillir ledit premier dispositif portable (1) ; et
- dans lequel le système est configuré pour effectuer une analyse répétée sur le conducteur même pendant la conduite et suite à la première analyse avant de monter dans le véhicule.

2. Système de mesure du BAC et/ou de la présence possible de substances actives illégales chez un conducteur (4) de véhicule selon la revendication 1, dans lequel ledit premier dispositif (1) s'interface avec ledit second dispositif (2) à l'intérieur du véhicule, et ledit second dispositif d'interface (2) est monté dans le véhicule en correspondance d'une zone du tableau de bord du véhicule étant accessible uniquement au conducteur (4) du véhicule pendant que ce dernier se trouve dans la condition assise de conduite.

3. Système de mesure du BAC et/ou de la présence possible de substances actives illégales chez un conducteur (4) de véhicule selon l'une quelconque des revendications précédentes, comprenant de plus un module de capteur de voix et d'interprétation pour le test vocal sur le conducteur (4).

4. Système de mesure du BAC et/ou de la présence possible de substances actives illégales chez un conducteur (4) de véhicule selon l'une quelconque des revendications précédentes, comprenant de plus une pluralité de dispositifs de détection (30) destinés à détecter la présence des mains du conducteur sur le volant (3).

5. Système de mesure du BAC et/ou de la présence possible de substances actives illégales chez un conducteur (4) de véhicule selon l'une quelconque des revendications précédentes, dans lequel ledit second dispositif d'interface (2) est disposé en correspondance d'une zone accessible uniquement au conducteur (4) pendant la conduite, ladite zone étant située en correspondance de la partie du tableau de bord comprise entre le volant (3) et la portière du véhicule.

6. Système de mesure du BAC et/ou de la présence possible de substances actives illégales chez un conducteur (4) de véhicule selon l'une quelconque des revendications précédentes, dans lequel le dispositif portable (1) s'interface avec le dispositif d'interface (2) par l'intermédiaire d'une technologie sans contact par un transpondeur.

7. Système de mesure du BAC et/ou de la présence possible de substances actives illégales chez un conducteur (4) de véhicule selon l'une quelconque des revendications 1-5, dans lequel le dispositif portable (1) s'interface avec le dispositif d'interface (2) par l'intermédiaire d'une technologie sans contact.

8. Utilisation d'un système de mesure du BAC et/ou de la présence possible de substances actives illégales chez un conducteur (4) de véhicule selon l'une quelconque des revendications précédentes, dans laquelle, pendant la conduite, le conducteur (4) interagit avec le premier dispositif portable (1) à travers une main et sans retirer ledit dispositif portable (1) du dispositif d'interface (2), et en gardant, en même temps, l'autre main en prise sur le volant (3).

9. Système de mesure du BAC et/ou de la présence possible de substances actives illégales chez un conducteur (4) de véhicule selon l'une quelconque des revendications 1-7, dans lequel ladite unité de commande est configurée pour effectuer le contrôle de la présence effective du conducteur (4) pendant l'analyse en vérifiant la présence de la main sur le volant (3) et l'autre main sur le dispositif mobile (1) .
